# EUROPÄISCHE PATENTANMELDUNG

(11) **EP 0 633 035 A1**
(43) Veröffentlichungstag der Anmeldung: **11.01.1995**
(21) Anmeldenummer: 94108406.3
(22) Anmeldetag: 01.06.1994
(51) Int. Cl.: A61M 5/172, A61M 5/168

(54) **Mehrkanal-Dosiersystem**

(30) Priorität: 19.06.1993 DE 4320365
(71) Anmelder: DRÄGERWERK AKTIENGESELLSCHAFT, D-23558 Lübeck (DE)
(72) Erfinder: Ebert, Holger, Prof., D-90429 Nürnberg (DE); Klinger, Bernt, Dr., D-23566 Lübeck (DE); Heimermann, Matthias, D-38302 Wolfenbüttel (DE); Hölscher, Uvo, Dr., D-23617 Stockelsdorf (DE)

(57) **Zusammenfassung**

Ein Mehrkanal-Dosiersystem (1) zur Dosierung von vorgewählten Fluidströmen aus einzelnen Fluidstromquellen (3), mit einer Steuereinrichtung (4), und mit in der Steuereinrichtung befindlichen, zumindestens die Dosierung der einzelnen Fluidströme beschreibenden Datenfeldern, welche auf eine mit der Steuereinrichtung verbundene Bedienoberfläche (5) mit einer Dateneingabeschaltung (10) und einer Datenausgabeschaltung (6) abrufbar sind, soll derart verbessert werden, daß das einer Fluidstromquelle zugeordnete Datenfeld einfach anwählbar ist. Zur Lösung der Aufgabe ist vorgesehen, daß einzelnen Fluidstromquellen (3) Auswahlschalter (13) als Teil der Dateneingabeschaltung (10) zugeordnet sind, durch welche zumindestens Segmente des zur angewählten Fluidstromquelle (3) gehörigen Datenfeldes auf der Bedienoberfläche (5) darstellbar sind, und durch welche zumindestens Teile der Dateneingabeschaltung (10) in Wirkverbindung mit dem zur angewählten Fluidstromquelle (3) gehörigen Datenfeld geschaltet sind.

## Beschreibung

Die Erfindung betrifft ein Mehrkanal-Dosiersystem zur Dosierung von vorgewählten Fluidströmen aus einzelnen Fluidstromquellen über separate, die Fluidströme aufnehmende Ablaufleitungen, mit den Fluidstromquellen zugeordneten, die Fluidströme beeinflussenden Stelleinrichtungen, welche mit einer programmierbaren Steuereinrichtung gekoppelt sind und mit in der Steuereinrichtung befindlichen, zumindestens die Dosierung der einzelnen Fluidströme beschreibenden Datenfeldern, welche auf eine mit der Steuereinrichtung verbundene Bedienoberfläche mit einer Dateneingabeschaltung und einer Datenausgabeschaltung abrufbar sind.

Ein Mehrkanal-Dosiersystem der genannten Art ist aus der EP-B 302 752 bekanntgeworden. Bei dem bekannten Dosiersystem sind an einem Fahrgestell eine Vielzahl von Infusionsbehältern als Fluidstromquellen befestigt und an einzelne Ablaufleitungen angeschlossen, durch welche die zu dosierenden Substanzen oder Fluidströme zu einer Sammelleitung gelangen, in der sich die Einzel-Fluidströme vereinigen. Im Leitungszug einer jeden Ablaufleitung befindet sich eine Durchfluß-Stelleinrichtung, mit der jeder Fluidstrom individuell beeinflußbar ist. Die Durchfluß-Stelleinrichtungen sind an eine zentrale, programmierbare Steuereinrichtung angeschlossen, welche sich im unteren Teil des Fahrgestells befindet, und welche die selektive Betätigung der einzelnen Durchfluß-Stelleinrichtungen gestattet. Die Steuereinrichtung besitzt eine Einrichtung zum Speichern von Informationen, in welcher Datenfelder über Abgabepläne der einzelnen Fluidarten abgelegt sind. Die Datenfelder sind in Form von Menübildschirmen auf eine Bedienoberfläche abrufbar, welche als Ausgabeeinrichtung einen "Touch Screen", zur Anzeige der einzelnen Menübildschirme oder Menümasken, und als Eingabeeinrichtung einen "Light Pen" besitzt, mit welchem über den Touch Screen einzelne Menüfunktionen aktivierbar sind. Mittels der Menümasken können einerseits die einzelnen Durchfluß-Stelleinrichtungen für die Fluidströme auf ausgewählte Dosierraten eingestellt werden, andererseits ist es möglich, Informationen über die Kompatibilität einzelner Fluidarten anzugeben, und es besteht über eine Keyboard-Menümaske die Möglichkeit, alphanumerische Daten, wie z. B. allgemeine Patientendaten oder Dosierraten, in die Steuereinrichtung einzulesen.

Das bekannte Dosiersystem ist von der Bedienungsseite her sehr komplex, da zur Realisierung von bestimmten Steuer- und Überwachungsfunktionen, unter Beachtung einer Bedienungshierachie, bestimmte Menümasken nacheinander aufgerufen werden müssen. Ein spontaner, unmittelbarer Zugriff auf das Datenfeld eines bestimmten Fluidstromes ist nicht möglich.

Aus der DE-A 33 29 977 ist ein Vierkanal Dosiersystem bekanntgeworden, bei welchem die Dosierraten der einzelnen Fluidströme von einer zentralen Steuereinrichtung überwacht werden, aber jeder Fluidstromquelle ein individueller Kodierschalter zugeordnet ist, mit welchem die Dosierrate von Hand eingestellt werden kann. Sämtliche Kodierschalter sind mit der Steuereinrichtung verbunden, die aus den von den Kodierschaltern an die Steuereinrichtung gelieferten Einzel-Dosierraten eine Summenförderrate errechnet und über ein Display an der Steuereinrichtung anzeigt.

Nachteilig bei dem bekannten Dosiersystem ist, daß aufgrund der dezentralen Dateneingabe für die einzelnen Dosierraten bei jeder Fluidstromquelle entsprechende Kodierschalter vorgehalten werden müssen, wodurch die Kodierschalter auch in einem für den Benutzer ungünstigen Blickfeld liegen können und die Gefahr einer Fehleinstellung groß ist.

Der Erfindung liegt die Aufgabe zugrunde, ein Mehrkanal Dosiersystem derart zu verbessern, daß einerseits wesentliche Bedienungsfunktionen auf einer zentralen Bedienoberfläche zusammengefaßt sind, andererseits die den einzelnen Fluidstromquellen zugeordneten Datenfelder einfach aufrufbar sind und bei der Bedienung die Gefahr der Verwechslung der zu dosierenden Fluide reduziert wird.

Die Lösung der Aufgabe erfolgt dadurch, daß einzelnen Fluidstromquellen und/oder vorbestimmten Gruppen von Fluidstromquellen Auswahlschalter als Teil der Dateneingabeschaltung zugeordnet sind, durch welche zumindestens Segmente des zur angewählten Fluidstromquelle gehörigen Datenfeldes, bzw. der zur angewählten Gruppe von Fluidstromquelle gehörigen Datenfelder auf der Bedienoberfläche darstellbar sind und durch welche zumindestens Teile der Dateneingabeschaltung in Wirkverbindung mit dem zur angewählten Fluidstromquelle gehörigen Datenfeld bzw. den zur angewählten Gruppe von Fluidstromquellen gehörigen Datenfeldern geschaltet sind.

Der Vorteil der Erfindung besteht im wesentlichen darin, daß mittels der Auswahlschalter ein unmittelbarer Zugriff auf das Datenfeld einer Fluidstromquelle bzw. die Datenfelder einer vorbestimmten Gruppe von Fluidstromquellen möglich ist. Die Anzahl der an ein Mehrkanal Dosiersystem angeschlossenen Fluidstromquellen kann größenordnungsmäßig bis zu zwanzig betragen, welche entweder gleichzeitig oder nach einer festgelegten Prioritätenfolge in Betrieb sind. Die Art der Zuordnung der Auswahlschalter entweder zu einzelnen Fluidstromquellen oder zu einzelnen Gruppen von Fluidstromquellen ergibt sich aus der Gesamtzahl der an das Dosiersystem angeschlossenen Fluidstromquellen. Bis zu einer Anzahl von etwa 6 Fluidstromquellen wird im allgemeinen jeder Fluidstromquelle individuell ein Auswahlschalter zugeordnet, und oberhalb dieser Grenze wird eine Gruppeneinteilung vorgenommen, wobei auch in jeder Gruppe die Obergrenze bei etwa sechs Fluidstromquellen liegt.

Wird mittels eines der Auswahlschalter das Datenfeld einer bestimmten Fluidstromquelle angewählt, kann die Wirkverbindung zwischen der Dateneingabeschaltung und dem Datenfeld in der Weise realisiert sein, daß ein unmittelbarer Zugriff auf Einstellparameter des angewählten Fluidstroms möglich ist. So kann z. B. die zum Fluidstrom gehörige Dosierrate an der Bedienoberfläche unmittelbar abgelesen und im Bedarfsfall auch dort verändert werden. Der Auswahlschalter ist im einfachsten Fall ein Drucktaster, welcher dem Benutzer bei der Betätigung eine taktile Rückmeldung gibt, daß der vorgewählte Fluidstrom an der Bedienoberfläche aktiviert ist.

Die Dateneingabe und Datenausgabe erfolgt über die Bedienoberfläche, welche hierzu eine Datenausgabeschaltung und eine Dateneingabeschaltung besitzt. Die Dateneingabeschaltung kann in Form von diskreten Drucktastern ausgeführt sein, mit denen Befehlsinformationen in die Steuereinrichtung eingegeben werden können. Zur Eingabe von analogen Größen, wie z. B. der Dosierrate, ist es zweckmäßig, ein kontinuierlich veränderbares Einstellglied mit einem Quittiertaster wie z. B. einen Drehknopf mit einem Winkelgeber und einem Druckkontakt als Quittiertaster, vorzusehen. Die Datenausgabeschaltung ist im einfachsten Fall ein LC-Display.

Vorteilhafte Ausgestaltungen der Erfindung sind in den Unteransprüchen angegeben.

In zweckmäßiger Weise sind die Auswahlschalter unmittelbar an den Fluidstromquellen und/oder den Stelleinrichtungen angeordnet. Hierdurch wird erreicht, daß der Benutzer zunächst bewußt eine bestimmte Fluidstromquelle durch Betätigung des Auswahlschalters anwählen muß, bevor eine Verstellung der Dosierrate über die Bedienoberfläche möglich ist. Ist beispielsweise die Fluidstromquelle als eine Spritze ausgeführt und die Stelleinrichtung ist ein den Spritzenkolben betätigender motorischer Antrieb, so ist der Auswahlschalter zweckmäßigerweise an der Spritze oder dem motorischen Antrieb angeordnet.

Es ist zweckmäßig, die Auswahlschalter mit einer die taktile Schalterbetätigung anzeigenden Indikatoreinrichtung zu versehen, welche beispielsweise als eine Leuchtdiode ausgeführt sein kann, die innerhalb des Tastenfeldes des Auswahlschalters angeordnet ist und bei Drucktastung des Auswahlschalters aufleuchtet. Auf diese Weise erhält der Benutzer eine direkte Rückmeldung, daß das zur ausgewählten Fluidstromquelle gehörige Datenfeld an der Bedienoberfläche aktiviert ist. Alternativ oder ergänzend zur optischen Anzeige kann auch ein akustischer Signalgeber vorhanden sein. In zweckmäßiger Weise ist jedem der Auswahlschalter jeweils eine Anzeigeeinheit zugeordnet, mit welcher ein vorbestimmter Fluidstromparameter dem Benutzer angezeigt wird. Der angezeigte Fluidstromparameter kann beispielsweise die zum jeweiligen Fluidstrom gehörige Dosierrate in Milliliter pro Stunde sein.

In einer vorteilhaften Ausgestaltung der Erfindung ist ein Handbetriebsschalter vorgesehen, durch welchen vorbestimmte Stelleinrichtungen in der Weise in die Abschaltlage bringbar sind, daß die Fluidstromdosierung manuell beeinflußbar ist. Bei Betätigung des Handbetriebsschalters wird durch die Steuereinrichtung ein Steuerbefehl erzeugt, durch welchen die vorbestimmte Stelleinrichtung in Abschaltlage geschaltet wird. Wurde der Handbetriebsschalter in Verbindung mit einem Auswahlschalter betätigt, so ist nur die zum Auswahlschalter gehörige Stelleinrichtung auf manuellen Betrieb geschaltet. Das Umschalten in die Abschaltlage kann auf folgende Weise realisiert sein. Ist beispielsweise die Stelleinrichtung als ein selbsthemmender, motorischer Antrieb mit einer Kupplung ausgeführt, so wird durch den Steuerbefehl der Kraftschluß zwischen Antrieb und Fluidstromquelle durch Betätigen der Kupplung aufgehoben. Sofern die Stelleinrichtung aus einem nicht selbsthemmenden Antrieb mit einer Bremse besteht, wird mit dem Steuerbefehl die Bremse entriegelt.

In einer zweckmäßigen Ausgestaltung der Erfindung ist zumindestens in der Abschaltlage der Stelleinrichtung eine Rückflußsperre in Funktion, mit welcher ein Rückstrom des Fluids von der Ablaufleitung in die Fluidstromquelle unterbunden wird. Die Rückflußsperre kann beispielsweise als ein Richtungsventil ausgeführt sein.

In einer vorteilhaften Ausgestaltung der Erfindung sind zur Überwachung der in die einzelnen Ablaufleitungen dosierten Fluidströme, z.B. im Handbetrieb, Durchflußmeßeinrichtungen vorgesehen, welche eine dem Durchfluß proportionale Meßgröße an die Steuereinrichtung liefern, welche dort mit gespeicherten Grenzwerten verglichen wird. Im Bedarfsfall wird von der Steuereinrichtung ein Warnsignal erzeugt und an der Bedienoberfläche zur Anzeige gebracht. Sollte die Dosierung des Fluidstroms auf Handbetrieb geschaltet sein, kann bei einer Grenzwertüberschreitung oder Grenzwertunterschreitung die manuelle Dosierung durch die Steuereinrichtung blockiert werden. Auf diese Weise kann auch eine Rückflußsperre realisiert sein. Die Durchflußmeßeinrichtungen können entweder mit den Stelleinrichtungen gekoppelt sein, oder sie sind als selbständige Einheiten den Fluidstromquellen nachgeschaltet. Eine Kopplung von Durchflußmeßeinrichtung und Stelleinrichtung liegt beispielsweise vor, wenn die Fluidstromquelle als eine Spritze und die Stelleinrichtung als ein den Spritzenkolben betätigender motorischer Antrieb ausgeführt ist und die Durchflußmeßeinrichtung die Wegänderung des Spritzenkolbens mißt. Ist die Durchflußmeßeinrichtung als ein das geförderte Flüssigkeitsvolumen messendes Bauglied ausgeführt, wird sie zweckmäßigerweise im Leitungszug einer Ablaufleitung angeordnet.

In einer vorteilhaften Ausgestaltung der Erfindung ist vorgesehen, das auf die Datenausgabeschaltung abrufbare Datenfeld in einzelne, den Fluidstromquellen zugeordnete sog. Primärsegmente und Sekundärsegmente zu strukturieren, wobei bei einer Statusänderung von Segmenten des Datenfeldes nur die Sekundärsegmente über die Auswahlschalter in Wirkverbindung mit der Dateneingabeschaltung gebracht werden, d.h. mittels der Dateneingabeschaltung verändert werden können. Primärsegmente des Datenfeldes sind beispielsweise Art und Ausführung der Fluidstromquelle, Spezifikation der zu dosierenden Lösung, das Körpergewicht des Probanden und die auf das Körpergewicht bezogene Dosierrate der zu dosierenden Lösung als Gewichtsdosis. Das Sekundärelement des Datenfeldes kann beispielweise die zur Gewichtsdosis gehörige Dosierrate in Volumen pro Zeiteinheit sein oder eine neu einzustellende Dosierrate vor ihrer Bestätigung durch den Benutzer.

In einer zweckmäßigen Ausgestaltung der Erfindung sind zumindestens einige der zu einer Fluidstromquelle gehörigen Primärsegmente und Sekundärsegmente in einer Anzeigezeile oder Anzeigespalte auf der Datenausgabeschaltung angeordnet. Der besseren Übersicht wegen ist es angebracht, unmittelbar miteinander korrespondierende Teile von Primärsegmenten und Sekundärsegmenten direkt übereinander oder nebeneinander in der Anzeige anzuordnen, wie z.B. die Gewichtsdosis und die Dosierrate. In Fällen, wo ein aktuelles Sekundärsegment viel Platz zur Anzeige benötigt, kann ein benachbartes Sekundärsegment kurzzeitig überschrieben werden. Die Primärsegmente sind von der Überschreibbarkeit ausgenommen.

In zweckmäßiger Weise ist auf der Datenausgebeschaltung ein Hinweisfeld vorgesehen, welches die Darstellung der Statusänderung zumindestens eines in Wirkverbindung mit der Dateneingabeschaltung gebrachten Sekundärsegmentes unterstützt. Dieses Sekundärsegment kann beispielsweise eine zur Änderung anstehende Dosierrate eines Fluidstroms sein, wobei im linken Teil des Hinweisfeldes die vorherige Dosierrate und im rechten Teil die neue Dosierrate angegeben ist. Im Hinweisfeld können auch Zusatzinformationen angezeigt werden, z.B. eine neu eingestellte Dosierrate zu überprüfen und zu bestätigen.

In einer zweckmäßigen Ausgestaltung der Erfindung ist vorgesehen, den Status von vorbestimmten Primärsegmenten in einer durch die Steuereinrichtung beeinflußten, zwangsgeführten Bedienungsfolge festzulegen. Diese zwangsgeführte Bedienungsfolge läuft beispielsweise ab, wenn Fluidstromquellen neu in das Dosiersystem eingelegt werden, und die Spezifikation der Fluidstromquelle, der zu dosierenden Lösung und Dosierparameter wie z.B. die Dosierrate in die Steuereinrichtung über die Dateneingabeschaltung eingegeben werden müssen, so daß keine Dosierung gestartet werden kann, ohne komplette Informationen vom Benutzer abgefragt zu haben.

In vorteilhafter Weise sind bei einer vorbestimmten Gruppe von Fluidstromquellen eines Mehrkanal-Dosiersystems den einzelnen Fluidstromquellen separat zugeordnete Anzeigeeinheiten vorgesehen, durch welche ein vorbestimmter Istwert des aus der jeweiligen Fluidstromquelle dosierten Fluidstroms zur Anzeige gebracht wird. Der vorbestimmte Istwert kann das von den Durchflußmeßeinrichtungen gemessene Fluidstrom-Volumen sein, womit dem Benutzer unmittelbar an jeder Fluidstromquelle das tatsächlich dosierte Fluidvolumen angezeigt wird und damit der angezeigte Wert auch visuell mit der zugehörigen Fluidstromquelle in Verbindung gebracht werden kann. Auch Unterbrechungen der Fluidstromdosierungen sind an den Anzeigeeinheiten unmittelbar ablesbar.

Ein Ausführungsbeispiel der Erfindung ist in der Zeichnung dargestellt und im folgenden näher erläutert.

Es zeigen:
- Fig. 1: den schematischen Aufbau eines Vierkanal Infusionssystems,
- Fig. 2: ein Beispiel für ein am LC-Display angezeigtes Datenfeld.
- Fig. 3: ein Beispiel für ein über das LC-Display dargestelltes Datenfeld bei einer Veränderung der Dosierrate,
- Fig. 4: eine nach Gruppen von Fluidstromquellen strukturierte Bedienoberfläche.

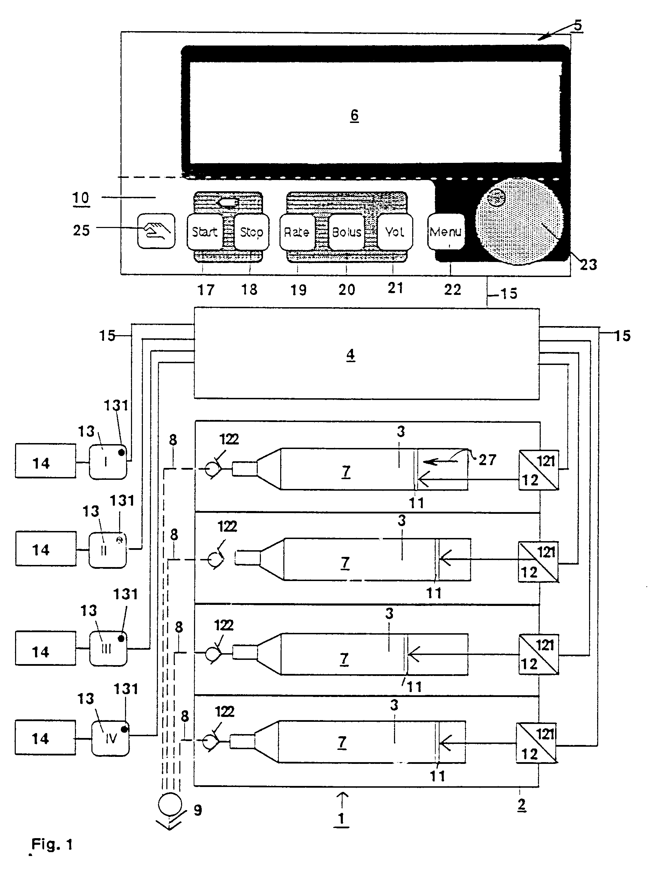

Das in der Figur 1 dargestellte Vierkanal-Dosiersystem (1) besteht aus einer Spritzenhalterung (2) für vier Dosierspritzen (3), einer Steuereinrichtung (4), und einer ersten Bedienoberfläche (5) mit einer Dateneingabeschaltung (10) und einem LC-Display (6) als Datenausgabeschaltung. Die Dosierspritzen, im folgenden als Spritzen (3) bezeichnet, sind mit Lösungen (7) als Fluide gefüllt, welche jeweils über Rückflußsperren (122) und Ablaufleitungen (8) an eine Sammelleitung (9) gelangen. Die Spritzen (3) dienen als Fluidstromquellen zur Dosierung von vorgewählten Fluidströmen, d.h. von Lösungen (7). Die Dosierung der Lösungen (7) erfolgt mittels in den Spritzen (3) befindlichen Spritzenkolben (11), welche jeweils durch einen motorischen Antrieb (12) als Stelleinrichtung hubbeweglich betätigt werden. Das dosierte Flüssigkeitsvolumen wird mittels Durchflußmeßeinrichtungen (121) erfaßt, welche die Wegänderungen der Spritzenkolben (11) registrieren. Neben jeder der Spritzen (3) befinden sich Auswahlschalter (13), mit denen einzelne Spritzen (3) angewählt werden können und eine Anzeigeeinheit (14), welche die jeweils eingestellte Dosierrate anzeigt. Die motorischen Antriebe (12), die erste Bedienoberfläche (5), die Durchflußmeßeinrichtungen (121), die Auswahlschalter (13) und die Anzeigeeinheiten (14) sind über Steuerleitungen (15) mit der Steuereinrichtung (4) verbunden. Die Steuereinrichtung (4) besitzt eine Einrichtung zum Speichern von Informationen, in welcher in der Figur 1 nicht dargestellte Datenfelder über Dosierpläne einer Vielzahl von zu dosierenden Lösungen abgelegt sind und eine ebenfalls nicht dargestellte Programmiereinrichtung, mit welcher Steuer- und Überwachungsfunktionen ausgeführt werden können. Die den einzelnen Lösungen zugeordneten Datenfelder enthalten beispielsweise die genaue Bezeichnung der Lösung, die Spritzenart und die Dosierrate.

Die Dateneingabeschaltung (10) dient zur Eingabe von Steuerbefehlen in die Steuereinrichtung (4) und besteht aus Drucktastern für Dosier-Start (17), Dosier-Stop (18), Dosierrate (19), Bolus (20), Dosiervolumen (21), Menü (22) und Handbetrieb (25), sowie einem analogen Einstellglied (23) mit integrierrem Quittiertaster, welcher durch Drucktastung des Einstellgliedes (23) betätigt wird. Die Bedienung des Dosiersystem (1) erfolgt durch das Zusammenwirken der Auswahlsschalter (13) mit den Drucktastern (17) bis (22) und dem Einstellglied (23). Mit den Auswahlschaltern (13) ist die Selektion eines bestimmten Kanals, d. h. einer bestimmten Lösung (7) möglich und über die Dateneingabeschaltung (10) können dann bestimmte Einstellungen innerhalb dieses ausgewählten Kanals vorgenommen werden. Die einzelnen Kanäle sind durch römische Ziffern I bis IV auf den Auswahlschaltern (13) markiert. Die Auswahlschalter sind mit Leuchtdioden (131) versehen, welche als Indikatoreinrichtung eine optische Rückmeldung der Schalterbetätigung geben.

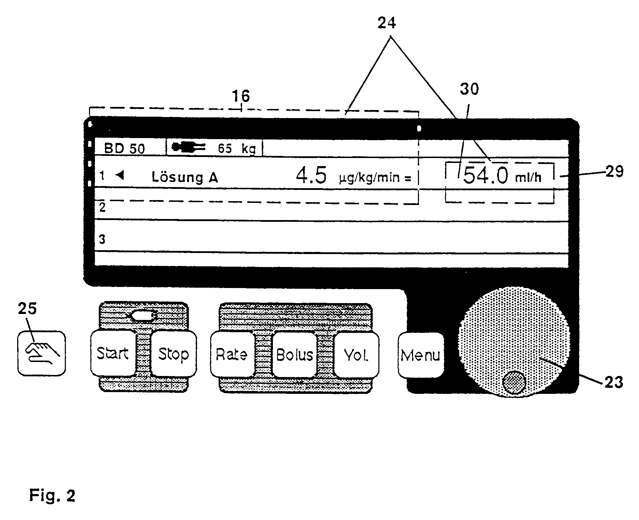

Figur 2 zeigt beispielhaft ein am LC-Display (6) angezeigtes Datenfeld (24) für die Festlegung einer Dosierrate einer zu dosierenden Lösung (7), Figur 1, im Kanal I.

Als Spritzentyp wurde eine Spritze (3) vom Typ "BD 50" mit der Lösung (7) "Lösung A" in die Spritzenhalterung (2) eingelegt, Figur 1. Der Proband hat ein Körpergewicht von 65 Kilogramm.

Das in Figur 2 gezeigte Datenfeld (24) ist in sogenannte Primärsegmente (16) und Sekundärsegmente (30) unterteilt. Die Primärsegmente (16) sind hier zusammengesetzt aus den Einzelsegmenten Spritzentyp "BD 50", Körpergewicht "65 kg", Kanalidentifikation "I", Spezifikation der Lösung "Lösung A" und der auf das Körpergewicht bezogenen Gewichtsdosis von 4,5 Mikrogramm/Kilogramm/Minute. Das dreieckförmige Symbol rechts neben der Kanalidentifikation "I" zeigt den Förderzustand Dosierung an.

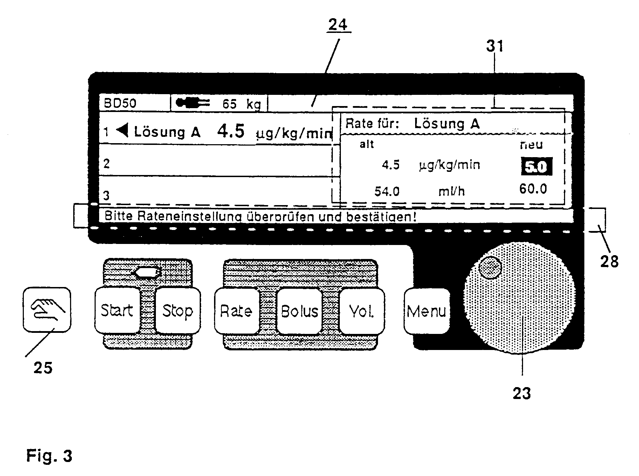

Das Sekundärsegment (30) gibt die Dosierrate "54.0 ml/h" an, wobei die korrespondierenden Segmente Gewichtsdosis und Dosierrate in einer Anzeigezeile (29) nebeneinander angeordnet sind. Soll nun die Dosierrate im Kanal "I" verändert werden, wird durch Druck auf den zum Kanal I gehörigen Auswahlschalter (13), Figur 1, der Kanal I angewählt und dann mittels des Einstellgliedes (23) durch Rechts- bzw. Linksdrehung die im Sekundärsegment (30) angezeigte Dosierrate erhöht oder erniedrigt. Bei der Anwahl des Kanals I überschreibt ein neues Sekundärsegment (31) das Sekundärsegment (30), wie dieses in der Figur 3 veranschaulicht ist. Das neue Sekundärsegment (31) erstreckt sich über drei Anzeigezeilen (29) und enthält gegenübergestellt die alte Dosierrate und die neue Dosierrate von 5.0 Mikrogramm/Kilogramm/Minute. Durch Druck auf das Einstellglied (23) wird die neue Dosierrate quittiert und in die Steuereinrichtung (4) eingelesen. In einer Hinweiszeile (28) des Datenfeldes (24) wird dazu aufgefordert, die Rateneinstellung zu überprüfen und zu bestätigen, was durch den vorherigen Druck auf das Einstellglied (23) bereits geschehen ist.

Der Bedienungsablauf des Dosiersystems bei Inbetriebnahme sei exemplarisch an einem Beispiel erläutert. Nach dem Einlegen und Verriegeln einer Spritze (3) in Kanal I wird automatisch das primäre Datensegment (16) (Figur 1, 2) des Kanals I aktiviert und es wird durch die Steuereinrichtung (4) eine zwangsgeführte Bedienungsfolge ausgelöst, die folgendermaßen abläuft. Über das LC-Display (6) erfolgt zunächst eine Aufforderung, den eingelegten Spritzentyp zu definieren. Hierzu werden eine Reihe von möglichen Spritzentypen angezeigt, danach der eingelegte Spritzentyp mittels des Einstellgliedes (23) ausgewählt und durch Druck auf das Einstellglied (23) quittiert. In gleicher Weise wird die in der Spritze (3) des Kanals I befindliche Lösung (7) ausgewählt. Im nächsten Schritt wird über das LC-Display (6) das Gewicht des Probanden abgefragt, sofern ein auf das Körpergewicht bezogener Dosiermodus gewählt wurde und zunächst ein voreingestellter Wert, von z. B. 65 Kilogramm, angezeigt, der durch Betätigung des Einstellgliedes (23) erhöht oder erniedrigt werden kann und zum Schluß durch Druck auf das Einstellglied (23) in die Steuereinrichtung (4) eingelesen wird. Im nächsten Schritt wird auf dem LC-Display (6) eine für die zu dosierende Lösung (7) und das Gewicht des Probanden empfohlene Dosierrate angezeigt, die mittels des Einstellgliedes (23) bei Bedarf ebenfalls erhöht oder erniedrigt werden kann und durch Druck auf das Einstellglied (23), d. h. Quittierung, bestätigt wird. Danach geht das Dosiersystem (1) in Standby-Funktion, und es können bei Bedarf in gleicher Weise weitere Kanäle definiert werden.

Zum Start der Dosierung wird zunächst der Kanal mit der zu dosierenden Lösung (7) angewählt, d. h. hier: Druck auf den Auswahlschalter (13) für Kanal I und danach Betätigung der Drucktaste Dosier-Start (17) (Figur 1). Soll während des Betriebs die Dosierrate verändert werden, muß zunächst der zugehörige Kanal durch Druck auf den Auswahlschalter (13) angewählt werden, danach Betätigung des Drucktasters Dosierrate (19) und Einstellen und Quittieren einer neuen Dosierrate mit dem Einstellglied (23). Durch Druck auf die Taste Dosier-Stop (18) kann die Dosierung in dem angewählten Kanal unterbrochen werden.

Neben einer durch die Steuereinrichtung (4) veranlaßten automatischen Dosierung von Lösungen (7) ist auch eine manuell gesteuerte Dosierung von Lösungen (7) möglich, welche folgendermaßen ausgeführt wird. Nach Auswahl des entsprechenden Fluidstrom-Kanals, z.B. Kanal I, mit dem zum Kanal I gehörenden Auswahlschalter (13) und anschließender Betätigung des Handbedienungsschalters (25), wird der zum Kanal I gehörige motorische Antrieb (12) durch einen von der Steuereinrichtung (4) abgegebenen Steuerbefehl in die Abschaltlage gebracht, so daß der Spritzenkolben (13) durch manuellen Druck längs des Pfeils (27) verschiebbar ist (Figur 1). Die Durchflußmeßeinrichtung (121) des Kanals 1 registriert die manuelle Kolbenverschiebung und liefert über die Steuerleitungen (15) entsprechende Meßsignale an die Steuereinrichtung (4), wo die dosierte Fluidrate überwacht wird und über das LC-Display (6) entsprechende Informationen hierüber dem Benutzer mitgeteilt werden. Die manuelle Dosierung bleibt so lange eingeschaltet, wie der Handbedienungsschalter (25) gedrückt wird. Beim Loslassen des Handbedienungsschalters (25) wird der motorische Antrieb (12) zunächst auf Standby geschaltet, bevor eine automatische Dosierung wieder aktiviert werden kann.

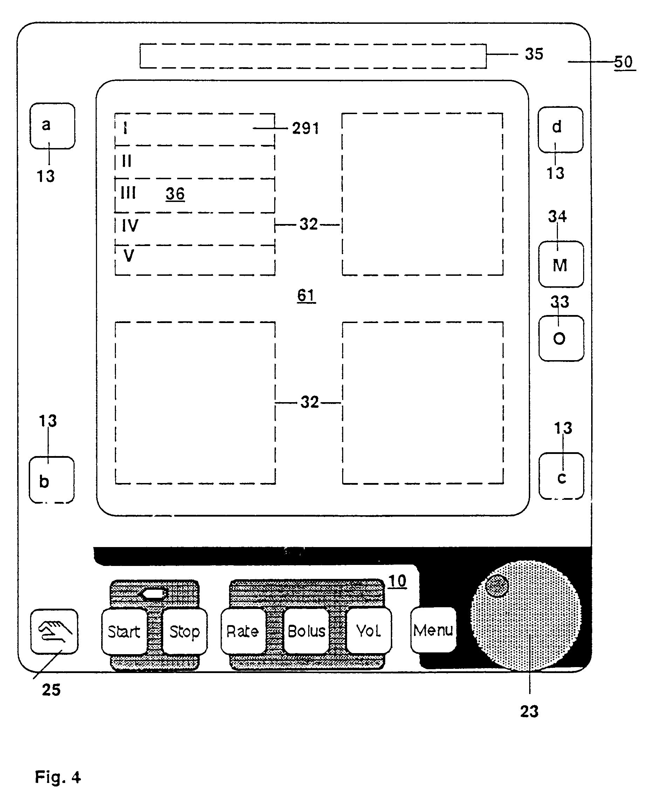

Figur 4 zeigt den schematischen Aufbau einer zweiten Bedienoberfläche (50) eines in der Figur nicht dargestellten Zwanzig-Kanal-Dosiersystems mit einem Bildschirm (61) als Datenausgabeschaltung und einer Dateneingabeschaltung (10) mit zugehörigen Auswahlschaltern (13) und Umschaltern (33, 34).

Gleiche Komponenten sind mit gleichen Bezugsziffern der Figuren 1 bis 3 bezeichnet. Unterschiedlich zu dem in der Figur 1 dargestellten Dosiersystem (1) ist, daß hier die Auswahlschalter (13, a, b, c, d) vorbestimmten Gruppen (32) von in der Figur 4 ebenfalls nicht dargestellten Fluidstromquellen (3) zugeordnet sind. Die Gruppen (32) sind auf dem Bildschirm (61) als gestrichelt gezeichnete Blöcke veranschaulicht und in der Gruppe 32 im linken oberen Teil des Bildschirms (61) ist, stellvertretend für die übrigen Gruppen, eine Kanalunterteilung durch römische Zahlen I bis V veranschaulicht mit Anzeigezeilen (291), welche jeweils einer bestimmten Fluidstromquelle (3) zugeordnet sind.

Die Struktur der Gruppen (32) kann entweder nach der physiologischen Wirkung der zu dosierenden Medikamente festgelegt sein, oder es werden organspezifische Gruppen gebildet, so daß durch die vorstrukturierten Gruppen bei einer Parameteränderung ein schneller Zugriff auf eine bestimmte Fluidstromquelle (3) möglich ist. Mittels des Umschalters (33) ist es möglich, ein organbezogenes Übersichtsschaubild in der Weise auf dem Bildschirm (61) darzustellen, daß die dosierten Lösungen (7) direkt dem betreffenden Organ zugeordnet sind. Mittels des Umschalters (34) ist ein Zurückschalten auf die medikamentenspezifische Darstellung möglich, welche in der Figur 4 veranschaulicht ist.

Soll auf der zweiten Bedienoberfläche (50) die Dosierrate einer bestimmten Fluidstromquelle, z.B. die des Kanals II, verändert werden, so wird zunächst mittels des Auswahlschalters (13, a) die zugehörige Gruppe (32) links oben im Bildschirm (61) angewählt und mittels eines über das Einstellglied (23) beeinflußbaren Cursors der Kanal II angewählt, und die Auswahl durch Druck auf das Einstellglied (23) quittiert.

Durch Überschreiben der den Kanälen zugeordneten in der Figur 4 nicht dargestellten Sekundärsegmente in den Anzeigezeilen (291) wird das ebenfalls nicht dargestellte Datenfeld des Kanals II in dem Feld (36) der linken oberen Gruppe (32) zur Anzeige gebracht und es kann in bekannter Weise die Dosierrate durch Drehen an dem Einstellglied (23) auf einen neuen Wert eingestellt werden. Im oberen Teil der Bedienoberfläche sind Anzeigevorrichtungen (35) für Warnungen vorgesehen, welche das Überschreiten von Grenzwerten signalisieren.

## Patentansprüche

1. Mehrkanal Dosiersystem (1) zur Dosierung von vorgewählten Fluidströmen aus einzelnen Fluidstromquellen (3) über separate, die Fluidströme aufnehmende Ablaufleitungen (8), mit jeweils den Fluidstromquellen zugeordneten, die Fluidströme beeinflussenden Stelleinrichtungen (12), welche mit einer programmierbaren Steuereinrichtung (4) gekoppelt sind und mit in der Steuereinrichtung (4) befindlichen, zumindestens die Dosierung der einzelnen Fluidströme beschreibenden Datenfeldern (24), welche auf eine mit der Steuereinrichtung verbundene Bedienoberfläche (5, 50) mit einer Dateneingabeschaltung (10) und einer Datenausgabesschaltung (6) abrufbar sind, dadurch gekennzeichnet, daß einzelnen Fluidstromquellen (3) und/oder vorbestimmten Gruppen (32) von Fluidstromquellen Auswahlschalter (13) als Teil der Dateneingabeschaltung (10) zugeordnet sind, durch welche zumindestens Segmente (16, 30, 31) des zur angewählten Fluidstromquelle (3) gehörigen Datenfeldes bzw. der zur angewählten Gruppe (32) von Fluidstromquellen gehörigen Datenfelder auf der Bedienoberfläche (5, 50) darstellbar sind, und durch welche zumindestens Teile der Dateneingabeschaltung (10) in Wirkverbindung mit dem zur angewählten Fluidstromquelle (3) gehörigen Datenfeld (24), bzw. den zur angewählten Gruppe (32) von Fluidstromquellen gehörigen Datenfeldern (24) geschaltet sind.

2. Dosiersystem nach Anspruch 1, dadurch gekennzeichnet, daß die Auswahlschalter (13) im Bereich der Fluidstromquellen (3) und/oder der Stelleinrichtungen (12) angeordnet sind.

3. Dosiersystem nach Anspruch 1 oder 2, dadurch gekennzeichnet, daß den Auswahlschaltern (13) eine die taktile Schalterbetätigung anzeigende Indikatoreinrichtung (131) zugeordnet ist.

4. Dosiersystem nach einem der Ansprüche 1 bis 3, dadurch gekennzeichnet, daß den Auswahlschaltern (13) einzelne, vorbestimmte Fluidstromparameter darstellende Anzeigeeinheiten (14) zugeordnet sind.

5. Dosiersystem nach einem der Ansprüche 1 bis 4, dadurch gekennzeichnet, daß ein Handbetriebsschalter (25) vorgesehen ist, durch welchen vorbestimmte Stelleinrichtungen (12) in der Weise in die Abschaltlage bringbar sind, daß die Fluidstromdosierung manuell beeinflußbar ist.

6. Dosiersystem nach Anspruch 5, dadurch gekennzeichnet, daß die vorbestimmte Stelleinrichtung (12) durch einen der Auswahlschalter (13) angewählt wird.

7. Dosiersystem nach einem der Ansprüche 1 bis 6, dadurch gekennzeichnet, daß Durchfluß-Meßeinrichtungen (121) zur Erfassung das in die jeweilige Ablaufleitung (8) dosierten Fluidstroms vorhanden sind, welche in Wirkverbindung zumindestens mit der Steuereinrichtung (4) stehen.

8. Dosiersystem nach einem der Ansprüche 1 bis 7, dadurch gekennzeichnet, daß das auf die Datenausgabeschaltung (6) abrufbare Datenfeld in einzelne, jeweils auf Fluidstromquellen (3) bezogene Primärsegmente (16) und Sekundärsegmente (30, 31) unterteilt ist, und daß zumindestens vorbestimmte Sekundärsegmente (30, 31) über die Auswahlschalter (13) zur Statusänderung in Wirkverbindung mit der Dateneingabeschaltung (10) bringbar sind.

9. Dosiersystem nach Anspruch 8, dadurch gekennzeichnet, daß zumindestens einige korrespondierende, zu einer Fluidstromquelle (3) gehörige Primärsegmente (16) und Sekundärsegmente (30) in einer Anzeigezeile (29) bzw. Anzeigespalte auf der Datenausgabeschaltung (6) dargestellt sind.

10. Dosiersystem nach Anspruch 8 oder 9, dadurch gekennzeichnet, daß die Datenausgabeschaltung (6) ein Hinweisfeld (28) zur Darstellung der Statusänderung zumindestens eines in Wirkverbindung mit der Dateneingabeschaltung (10) gebrachten Primärsegmentes (16) und/oder Sekundärsegmentes (30) besitzt.

11. Dosiersystem nach einem der Ansprüche 8 bis 10, dadurch gekennzeichnet, daß der Status zumindest einzelner Teile von Primärsegmenten (16), in einer durch die Steuereinrichtung (4) vorbestimmten, zwangsgeführten Bedienungsfolge festgelegt ist.

12. Mehrkanal-Dosiersystem (1) zur Dosierung von vorgewählten Fluidströmen aus einzelnen Fluidstromquellen (3) über separate, die Fluidströme aufnehmende Ablaufleitungen (8), mit jeweils den Fluidstromquellen zugeordneten, die Fluidströme beeinflussenden Stelleinrichtungen (12), welche mit einer programmierbaren Steuereinrichtung (4) gekoppelt sind und mit in der Steuereinrichtung befindlichen, zumindestens die Dosierung der einzelnen Fluidströme beschreibenden Datenfeldern (24), welche auf eine mit der Steuereinrichtung verbundene Bedienoberfläche (5, 50) mit einer Dateneingabeschaltung (10) und einer Datenausgabeschaltung (6) abrufbar sind, dadurch gekennzeichnet, daß eine vorbestimmte Gruppe (32) von Fluidstromquellen (3) mit einem Auswahlschalter (13) als Teil der Dateneingabeschaltung (10) versehen ist, durch welchen zumindestens Segmente (16, 30, 31) der zur vorbestimmten Gruppe (32) der Fluidstromquellen (3) gehörigen Datenfelder (24) auf die Bedienoberfläche (5, 50) bringbar sind, und daß separate, den Fluidstromquellen (3) oder den Stelleinrichtungen (12) zugeordnete Anzeigeeinheiten (14) vorgesehen sind, durch welche ein vorbestimmter Istwert des aus der jeweiligen Fluidstromquelle (3) dosierten Fluidstroms zur Anzeige gebracht wird.
